# EUROPEAN PATENT SPECIFICATION

(11) **EP 3 023 941 B1**
(45) Date of publication and mention of the grant of the patent: **08.05.2019**
(21) Application number: 15192812.4
(22) Date of filing: 17.03.2010
(51) Int. Cl.: G06T 15/00

(54) **SYSTEM FOR PROVIDING VISUAL GUIDANCE FOR STEERING A TIP OF AN ENDOSCOPIC DEVICE TOWARDS ONE OR MORE LANDMARKS AND ASSISTING AN OPERATOR IN ENDOSCOPIC NAVIGATION**
VORRICHTUNG ZUR BEREITSTELLUNG VON VISUELLER FÜHRUNG ZUM LENKEN EINER SPITZE EINER ENDOSKOPISCHEN VORRICHTUNG ZU EINER ODER MEHREREN LANDMARKEN UND UNTERSTÜTZUNG EINES BEDIENERS BEI DER ENDOSKOPISCHEN NAVIGATION
SYSTÈME POUR FOURNIR UN GUIDAGE VISUEL POUR DIRIGER L'EMBOUT D'UN DISPOSITIF ENDOSCOPIQUE VERS UN OU PLUSIEURS REPÈRES ET ASSISTER UN OPÉRATEUR LORS D'UNE NAVIGATION ENDOSCOPIQUE

(30) Priority: 26.03.2009 US 411501; 26.03.2009 US 411515
(43) Date of publication of application: 25.05.2016
(62) Divisional of application: 10711112.2
(73) Proprietor: Intuitive Surgical Operations, Inc., Sunnyvale, CA 94086 (US)
(72) Inventor: PRISCO, Giuseppe, 56011 Calci, Pisa (IT); DUINDAM, Vincent, Sunnyvale, CA 94086 (US)
(74) Representative: MacDougall, Alan John Shaw

(56) References cited:
- WO-A1-2007/036925
- CN-A- 101 026 988
- US-A1- 2002 062 062
- US-A1- 2005 182 295
- US-A1- 2007 197 896
- US-A1- 2007 293 721

## Description

### FIELD OF THE INVENTION

The present invention generally relates to steerable endoscopes and in particular, to a system for providing visual guidance for steering a tip of an endoscopic device towards one or more landmarks and assisting an operator in endoscopic navigation.

### BACKGROUND OF THE INVENTION

Minimally invasive surgical procedures are known to offer many benefits over traditional open surgery techniques, including less pain, shorter hospital stays, quicker return to normal activities, minimal scarring, reduced recovery time, and less injury to tissue. To perform such procedures, entry into the patient may be made through an incision or a natural body orifice. Both robotic and manually operated minimally invasive surgical devices may be used.

One example of a minimally invasive surgical robotic system is the da Vinci® Surgical System from Intuitive Surgical, Inc., of Sunnyvale, California. The da Vinci® Surgical System has a number of robotic arms that move attached medical devices, such as an image capturing device and Intuitive Surgical's proprietary EndoWrist® articulating surgical instruments, in response to movement of input devices by a surgeon viewing images captured by the image capturing device at a surgical site. Each of the medical devices may be inserted through its own minimally invasive incision into the patient and positioned to perform a medical procedure at the surgical site. The incisions are placed about the patient's body so that the surgical instruments may be used to cooperatively perform the medical procedure and the image capturing device may view it without their robotic arms colliding during the procedure.

An endoscope is a medical device that allows physicians to capture images of and diagnose problems with internal body organs by inserting the device either through a natural orifice or a surgeon created opening and guiding it to a target site within a patient. In some cases, it may also be used to perform medical procedures on the internal body organs. It may be steerable so that its distal tip is controllably oriented for navigation purposes. An image capturing device such as a stereoscopic or monoscopic camera may be provided at its distal tip so that images captured by the camera from that perspective may be viewed on a display screen by the surgeon. To perform various medical procedures at the target site, surgical tools, such as those used for cutting, grasping, cauterizing, etc., may extend out of the endoscope's distal tip.

The endoscope may be rigid such as those used in laparoscopy or it may be flexible so that it is capable of following the curvatures of body lumens. It may also be rigidizable and/or robotic. A rigidizable endoscope is an endoscope that has at least one section of its flexible body that can be made substantially rigid by a mechanical locking mechanism. A robotic endoscope is a flexible endoscope that has at least one section that bends under a computer controlled servo mechanism. It may also be a capsule like the EndoCapsule by Olympus or a tethered capsule in which case it is not controllably oriented for navigation purposes and gets moved in the patient anatomy by gravity, forces applied by the anatomy or by other surgical devices.

Natural Orifice Transluminal Endoscopic Surgery ("NOTES") may employ a steerable endoscope to perform surgical procedures on patients. As an example, a flexible endoscope may be guided through one of the body's orifices and enter the abdomen from the inside of the patient, rather than through a minimally invasive incision from the outside. For example, in "transgastric" surgery, instruments are passed through the mouth and into the stomach. A gastrotomy is then performed so that the instruments may enter the abdomen and be used by the surgeon to perform a medical procedure within the abdominal cavity. Once the procedure is completed, the instruments are withdrawn along with any tissue removed during the procedure, and the entry point is closed back up. Because no incisions are made in the patient to accommodate entry of the endoscope, NOTES may be even less painful than surgery using minimally invasive incisions. Also, since it uses a natural body orifice instead of incisions to enter the body, it may result in reduced needs for general anesthetics and faster recovery times.

During the operation of a steerable endoscope, such as in a NOTES application, the endoscope tip may be turned multiple times and in different directions while moving towards a target site. As a consequence, the flexible endoscope may wind up looping around itself and disorienting the operator so as to make it difficult for the operator to keep track of the current direction of the endoscope tip if its captured image fails to clearly indicate its current direction with respect to the target site. In contrast, the operator may have a relatively rigid connection to the view in conventional laparoscopy.

If the operator accidentally moves the endoscope tip in the wrong direction, the tip may inadvertently perforate or otherwise damage tissue causing harm to the patient. Even if such harm is avoided by carefully moving the endoscope tip, additional time is required to repeatedly ascertain the true direction of the endoscope relative to the target site in the patient. Thus, the time required to perform the procedure is lengthened which adds to the cost of the surgery and increases health safety concerns.

US 20020062062 A1 discloses a steerable endoscope having an elongated body with a selectively steerable distal portion and an automatically controlled proximal portion. The endoscope body is inserted into a patient and the selectively steerable distal portion is used to select a desired path within the patient's body. When the endoscope body is advanced, an electronic motion controller operates the automatically controlled proximal portion to assume the selected curve of the selectively steerable distal portion. Another desired path is selected with the selectively steerable distal portion and the endoscope body is advanced again. As the endoscope body is further advanced, the selected curves propagate proximally along the endoscope body, and when the endoscope body is withdrawn proximally, the selected curves propagate distally along the endoscope body.

US 20070197896 A1 discloses a method for performing an interventional procedure using a robotically controlled guide instrument coupled to an instrument drive assembly, includes manipulating a user interface of a master controller to actuate the drive assembly and thereby position a distal portion of the guide instrument at an interventional procedure site in a patient's body; and providing on a first display a graphically rendered image of the distal portion of the guide instrument overlaying an image of the interventional procedure site, wherein manipulation of the user interface causes a corresponding movement of the instrument model relative to the image in the first display.

WO 2007036925 A1 discloses an apparatus for treating a lumen, comprising: at least one mapping tool, wherein the at least one mapping tool is adapted and constructed to provide at least a partial map of an internal surface of the lumen; a display, adapted and constructed to permit the identification of at least one point of interest in the lumen, using the at least partial map; at least one sensor, wherein data retrieved from the at least one sensor is compared to the at least partial map for navigating at least one procedural instrument within the lumen; and, wherein treatment by the at least one procedural instrument occurs when sensed data compared with the at least partial map indicates the that least one procedural instrument is at at least one point of interest.

### OBJECTS AND SUMMARY OF THE INVENTION

The present invention provides a system comprising: a display; an input device; an endoscopic device having a flexible body, steerable tip, and electromechanical interface; an image capturing device positioned at a distal end of the steerable tip of the endoscopic device so as to capture images from a perspective of the distal end; and a memory device; and one or more processors adapted to control bending of the flexible body and steering of the steerable tip of the endoscopic device in response to manipulation of the input device, reference a computer model of a patient to a reference frame, determine a current position and shape of the endoscopic device relative to the reference frame, generate a computer model of the endoscopic device according to the determined current position and shape, capture a plurality of images with the image capturing device, store the plurality of captured images in the memory device, determine and store positions of the image capturing device at the times of such image capturing and display the computer models of the endoscopic device and patient on the display and a synthetic view generated by assembling the plurality of captured images into a panoramic image by mosaicing the plurality of images together so as to provide endoscopic navigation assistance to an operator of the system.

Also disclosed is a system comprising: a display screen; an endoscopic device; and one or more processors adapted to determine a current position of a reference point on the endoscopic device relative to a reference frame, determine a first vector from the current position of the reference point to a position of a first landmark relative to the reference frame, transform the first vector from the reference frame to an image reference frame that corresponds to the perspective of an image capturing device disposed at a tip of the endoscopic device, and display a graphical representation of the transformed first vector along with a current image captured by the image capturing device on a display screen so as to indicate a direction to steer the tip of the endoscopic device towards the first landmark.

Also disclosed, but not being part of the invention, is a computer implemented method for assisting an operator in endoscopic navigation, the method comprising: referencing a computer model of a patient to a reference frame; determining a current position and shape of an endoscopic device relative to the reference frame; generating a computer model of the endoscopic device according to the determined current position and shape; and displaying the computer models of the endoscopic device and patient on a display screen so as to provide the endoscopic navigation assistance to the operator.

Also disclosed is a system comprising: a display screen; an endoscopic device; and a processor adapted to reference a computer model of a patient to a reference frame, determine a current position and shape of the endoscopic device relative to the reference frame, generate a computer model of the endoscopic device according to the determined current position and shape, and display the computer models of the endoscopic device and patient on the display screen so as to provide navigation assistance to an operator of the system.

Additional objects, features and advantages of the various aspects of the present invention will become apparent from the following description of its preferred embodiment, which description should be taken in conjunction with the accompanying drawings.

### BRIEF DESCRIPTION OF THE DRAWINGS

**FIG. 1** illustrates a steerable endoscope system utilizing aspects of the present invention.
**FIG. 2** illustrates a block diagram of a processing portion of a steerable endoscope system utilizing aspects of the present invention.
**FIG. 3** illustrates a block diagram of a teleoperative portion of a steerable endoscope system utilizing aspects of the present invention.
**FIG. 4** illustrates a flow diagram of a computer implemented method for assisting an operator in endoscopic navigation, utilizing aspects of the present invention.
**FIG. 5** illustrates parts of a position determining portion of a steerable endoscope system utilizing aspects of the present invention.
**FIG. 6** illustrates a primary display screen displaying an image captured by a steerable endoscope as viewed in a system utilizing aspects of the present invention.
**FIG. 7** illustrates an auxiliary display screen displaying an anterior-posterior view of registered patient and endoscopic device models as generated by a system utilizing aspects of the present invention.
**FIG. 8** illustrates an auxiliary display screen displaying an oblique view of patient and endoscopic device models as generated by a system utilizing aspects of the present invention.
**FIG. 9** illustrates an auxiliary display screen displaying a lateral-medial view of patient and endoscopic device models as generated by a system utilizing aspects of the present invention.
**FIG. 10** illustrates a primary display screen displaying an image captured by an endoscopic device in a main window area and an oblique view of patient and endoscopic device models in a picture-in-picture window area as generated by a system utilizing aspects of the present invention.
**FIG. 11** illustrates a primary display screen displaying an image captured by an endoscopic device in a first area and an oblique view of patient and endoscopic device models in a second area as generated by a system utilizing aspects of the present invention.
**FIG. 12** illustrates a flow diagram of a computer implemented method of providing directional guidance to maneuver an endoscopic device to one or more landmarks, utilizing aspects of the present invention.
**FIG. 13** illustrates a flow diagram of a computer implemented method for establishing landmarks using an endoscopic device as it moves by the landmarks, utilizing aspects of the present invention.
**FIG. 14** illustrates an oblique view of a patient torso with an endoscopic device inserted therein and examples of various landmarks as employed in a system utilizing aspects of the present invention.
**FIG. 15** illustrates a primary display screen displaying a current image captured by an endoscopic device and graphical representations of various vectors indicating directions to corresponding landmarks along with an image associated with one of the landmarks, according to a method utilizing aspects of the present invention.

### DETAILED DESCRIPTION OF THE PREFERRED EMBODIMENT

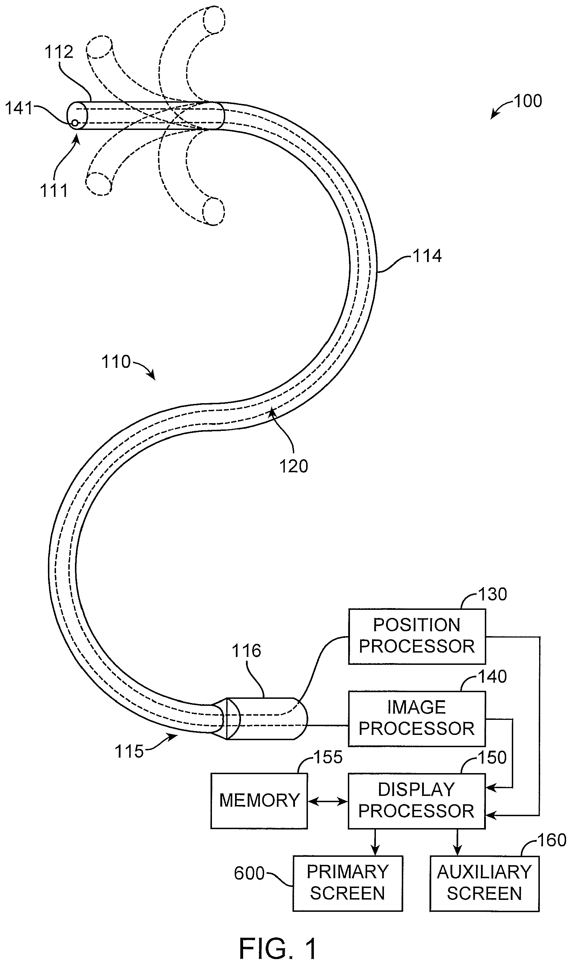

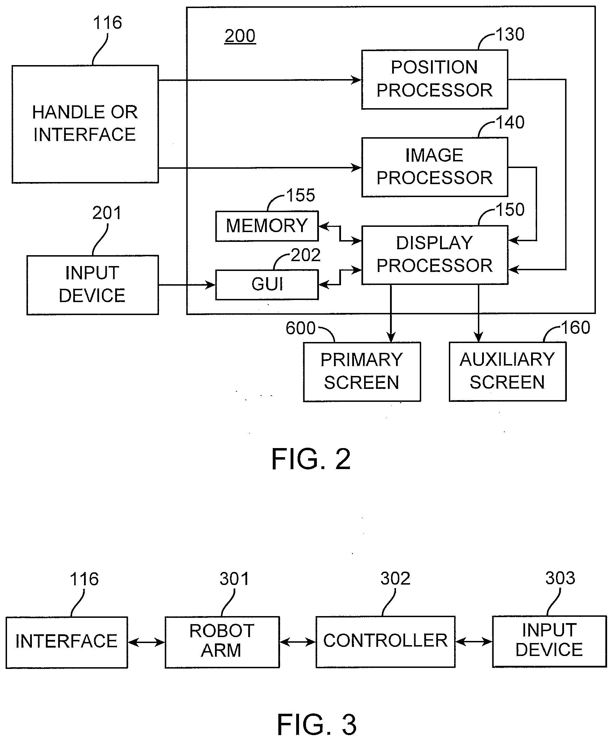

**FIG. 1** illustrates, as an example, a system **100** including a steerable endoscope **110**, one or more fiber optic cables **120** inserted in the endoscope **110**, a position processor **130**, an image processor **140**, an image capturing device **141**, a display processor **150**, a primary display screen **600**, and an auxiliary display screen **160**. Although shown as separate units, the position processor **130**, image processor **140** and display processor **150** may each be implemented as hardware, firmware, software or a combination thereof, which interact with or are otherwise executed by one or more computers, such as the one or more computers **200** depicted in **FIG. 2**. The primary and auxiliary display screens, **600** and **160,** are preferably computer monitors capable of displaying three-dimensional images to an operator of the system **100**. However, for cost considerations, either or both of the primary and auxiliary display screens, **600** and **160**, may be a standard computer monitor capable of only displaying two-dimensional images.

The endoscope **110**, in this example, has a flexible body **114**, a steerable tip **112** at its distal end **111** and a handle or electromechanical interface **116** at its proximal end **115**. Control cables (not shown) or other control means typically extend from the handle or electromechanical interface **116** to the steerable tip **112** so that the tip **112** may be controllably bent or turned as shown for example by dotted line versions of the bent tip **112**. Although a steerable endoscope is described in this example, the present invention is not to be limited to endoscopes of this type and may be practiced with other endoscopic devices (such as rigid, rigidizable, robotic, or capsule endoscopes) as well.

A stereoscopic or monoscopic camera **141** is provided at the distal end **111** for capturing images that are transmitted to and processed by the image processor **140** and/or display processor **150** and displayed on the primary display screen **600** and/or auxiliary display screen **160** according to the various aspects of the invention as described herein. One of a plurality of fiber optic cables **120** may be coupled at its proximal end to a light source (not shown) for illumination purposes at the distal end **111**. Others of the fiber optic cables **120** may be configured with bend or shape sensors such as Fiber Bragg Gratings (or other strain sensors such as those employing Rayleigh scattering) so that light passing through the fiber optic cable is processed by the position processor **130** to determine a current position and shape of the endoscope **110** including the orientation of its distal tip **112**. In addition to the fiber optic cables **120** extending through the endoscope **110**, one or more additional fiber optic cables (not shown) configured with strain sensors may be attached to the endoscope **110** so as to provide position information of the endoscope **110** at the attachment point.

When the steerable endoscope **110** is manipulated manually by an operator, a handle **116** is used with appropriate levers or other control mechanism(s) for controllably orienting the endoscope tip **112** by pulling appropriate cables (not shown) which couple such control mechanisms of the handle **116** to the tip **112**. On the other hand, when the steerable endoscope **110** is manipulated teleoperatively by an operator, an electromechanical interface **116** is used instead of a handle.

As shown in **FIG. 3**, the electromechanical interface **116** allows the endoscope **110** to be electrically and mechanically coupled to a robot arm **301** whose movement is controlled by a controller **302** in response to operator manipulation of an input device **303**. Either or both the interface **116** and the robot arm **301** include motors that drive the cables used to steer the endoscope tip **112**. In addition to controlling bending of the endoscope's tip **112**, the robot arm **301** may also be configured to insert/retract the endoscope **110** into and out of an aperture (e.g., natural orifice or minimally invasive incision) in the patient, rotate the endoscope **110** about its central axis, and/or rotate the electromechanical interface **116** about a pivot point at the aperture. The controller **302** is preferably implemented as hardware, firmware or software (or a combination thereof) in the one or more computers **200** along with the processors **130**, **140**, **150**.

Details on the determination of the endoscope's position and bending using Fiber Bragg Gratings may be found, for examples, in U.S. 2007/0156019 A1 entitled "Robotic Surgery System Including Position Sensors Using Fiber Bragg Gratings", U.S. 2008/0212082 A1 entitled "Fiber Optic Position and/or Shape Sensing Based on Rayleigh Scatter", U.S. 2008/0218770 A1 entitled "Robotic Surgical Instrument and Methods using Bragg Fiber Sensors", and U.S. Applic. Serial No. 12/164,829 entitled "Fiber Optic Shape Sensor. Details on a conventional steerable endoscope may be found for example in U.S. 6,869,396 B2 entitled "Steerable Endoscope and Improved Method of Insertion".

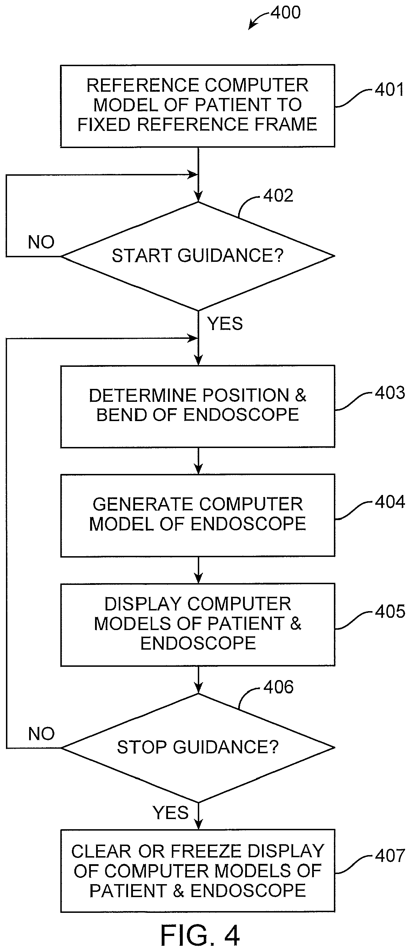

**FIG. 4** illustrates, as an example, a flow diagram of a computer implemented method **400** for assisting an operator in endoscopic navigation of the steerable endoscope **110** towards a site in a patient. The computer in this case is preferably the one or more computers **200** that include the processors **130**, **140**, **150**.

In **401**, a patient computer model stored in memory **155** is referenced to a fixed reference frame (i.e., one that does not move during the medical procedure to be performed using the endoscope **110**) by first referencing points on a live patient who is generally prone on an operating table with the fixed reference frame, then associating the referenced points with corresponding points on the patient computer model.

For example, using a pointer device whose tip position may be readily determined in the fixed reference frame (such as the endoscope's tip **112**), the operator may move the pointer device so that it first touches the mouth of the patient to establish a first reference point corresponding to the mouth of the patient computer model. The operator may then move the pointer device so that it next touches the right and left crest of the iliac of the patient to establish a second reference point corresponding to the right and left crest of the iliac of the patient computer model. Other points such as the navel and the left and right arm pits may also be used to establish more reference points. By referencing the patient computer model to the patient in this manner, the proper size (i.e., length and width) of the patient computer model may also be established as well as its alignment with the patient and its position in the fixed reference frame. Thus, with just a few points, the patient computer model may be properly scaled and aligned (i.e., conformed) with the patient using model information in an anthropometry database in the memory **155**.

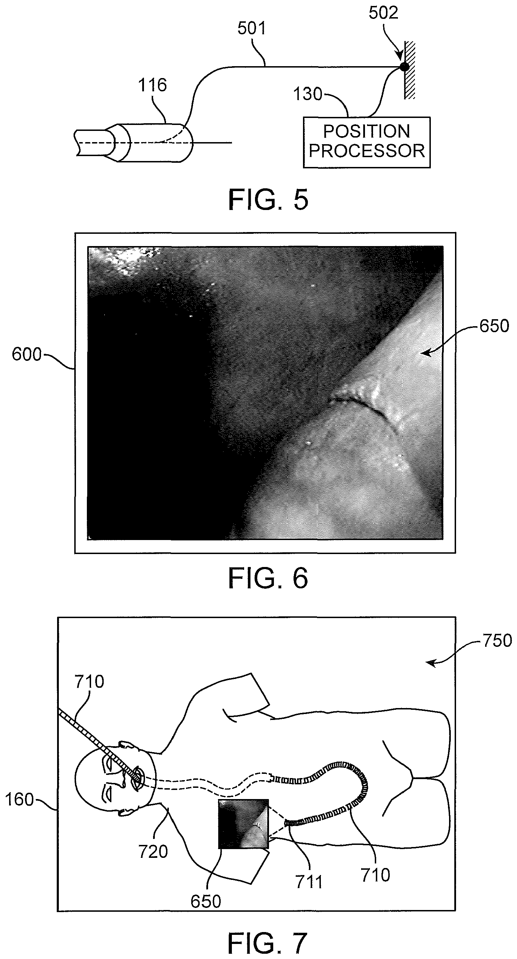

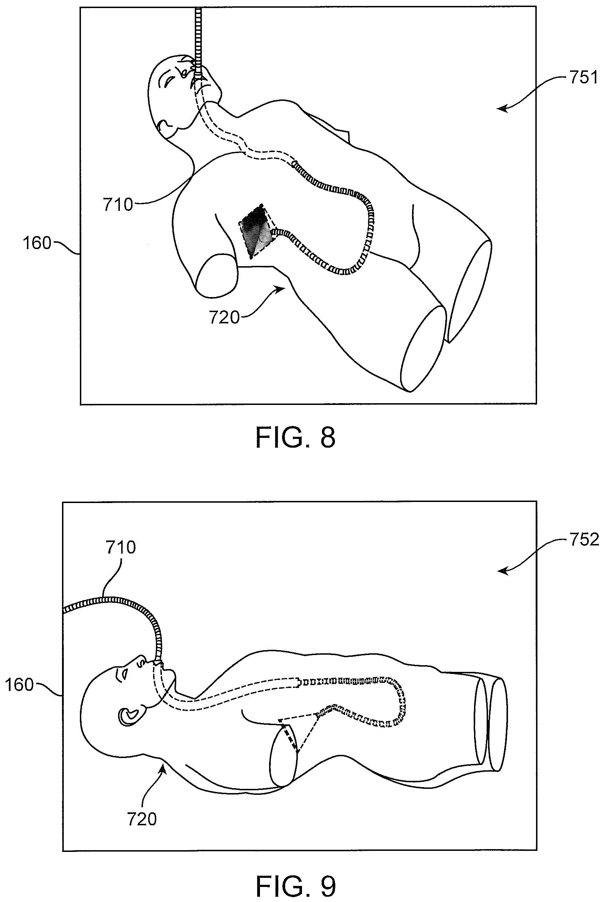

Several patient computer models may be stored in memory **155**. For example, a number of generic patient computer models may be stored so that a different model may be used depending on the age, sex, size, etc., of the patient. Preferably, each of the models is a three-dimensional computer model including at least a head and torso (such as seen in **FIGS. 7-9**) so that all natural orifices are included as well as potential target sites for performing diagnostic or surgical procedures on a patient. Three-dimensional computer models of the actual patient may also be stored and used with the present system if available, such as may be generated using a Computed Tomography (CT) scan. Alternatively, the surgeon or assistant may move the distal tip **112** over the surface of the patient's body to map its profile and provide sufficient information to the position processor **130** to generate information for a three-dimensional computer model of the patient which it stores in the memory **155**.

In **402**, a determination is made whether or not the operator has issued a start guidance command. The operator may issue the start guidance command in any one of a number of ways such as depressing an input button or flipping a switch on the input device **303** when the steerable endoscope **110** is being controlled teleoperatively, or depressing an input button or flipping a switch on the handle **116** when the steerable endoscope **110** is being controlled manually. Other ways may include voice actuation using a microphone and voice recognition processor (not shown) that may be implemented in or operatively coupled to the one or more computers **200**; a foot pedal (not shown) that may be depressed by the operator so as to turn on or off the guidance; and a graphical user interface using the auxiliary display screen **160**, the primary display screen **600** or another display screen (not shown) that the operator may interact with using an input device.

If the determination in **402** is NO (i.e., navigation guidance mode has not been initiated by the operator), then the method loops through **402** each process cycle (or other programmed cycle) until a YES determination is made, at which time, the method proceeds to **403**.

In **403**, the current position and shape (e.g., bending) of the endoscope **110** is determined by the position processor **130** using information received from one or more optical fibers configured with shape sensors that has been inserted in the flexible body **114** of the endoscope **110**. As an example of one such optical fiber, referring to **FIG. 5**, the distal end of an optical fiber **501** extends to the endoscope's tip **112** and its proximal end is attached to a base point **502** whose position in the fixed reference frame is known or readily determinable by the position processor **130**. Preferably, the position processor **130** is also attached or otherwise coupled to the base point **502** so that the processor **130** may receive information from the bend sensors of the optical fiber **501** at that point.

Since the length of the fiber optic cable **501** is known, its proximal end is fixed to a known location, and its distal end extends out to the endoscope's tip **112**, the current position and orientation of the tip **112** in the fixed reference frame may be readily determined from the current bending of the optical fiber **501** as indicated by information received from the bend sensors. Although only one fiber optic cable **501** is shown for illustrative purposes, multiple fibers or a single fiber with multiple cores configured with strain sensors are preferably employed for determining the shape of the endoscope body **114** and distal tip **112**, such as described using shape sensors in U.S. 2007/0156019 A1, U.S. 2008/0212082 A1, and U.S. Application Serial No. 12/164,829.

In **404**, a computer model that is indicative of the current configuration of the flexible endoscope **110** is generated using a model of the endoscope stored in the memory **155** and modifying it to reflect the shape information received from the optical fibers configured as shape sensors. Preferably, both the stored and generated computer models of the endoscope **110** are three-dimensional models.

In **405**, the patient computer model generated in **401** and the endoscope computer model generated in **404** are displayed together properly registered with respect to each other according to their respective positions in the fixed reference frame on the auxiliary display screen **160**. Thus, the operator of the steerable endoscope **110** is provided a view of the current shape of the endoscope **110** relative to the patient's body in order to provide guidance to the operator for navigating the endoscope **110** to a target site within the patient. A visual indication of the target site may also be displayed as well as computer models or other indications of any anatomic structures or body lumens that the endoscope **110** may encounter or pass through in its path to the target site.

The manner in which the patient and endoscope computer models are displayed on the auxiliary display screen **160** is preferably selectable by the operator using any one or more of a number of well known techniques such as clickable icons and pull down menus provided on the auxiliary display screen **160** and/or primary display screen **600**. For example, icons may be provided on the auxiliary display screen **160** so that the operator may select an anterior-posterior view **750**, oblique view **751**, or lateral-medial view **752** of the patient and endoscope **110**, such as shown respectively in **FIGS. 7**, **8** and **9**, by clicking on the appropriate icon using a computer mouse or other suitable device.

Referring to **FIG. 7**, the anterior-posterior view **750** of the patient computer model **720** and endoscope computer model **710** is shown along with indications of various landmarks along the endoscope's path (e.g., mouth entry, esophagus, stomach entry, and colon) on the auxiliary display screen **160**. In addition, real-time images captured by the endoscope **110** (such as the captured image **650** shown in **FIG. 6** being displayed on a primary display screen **600**) may also be displayed on the auxiliary display screen **160** adjacent to (or appearing to emanate from) the distal end **711** of the endoscope computer model **710**.

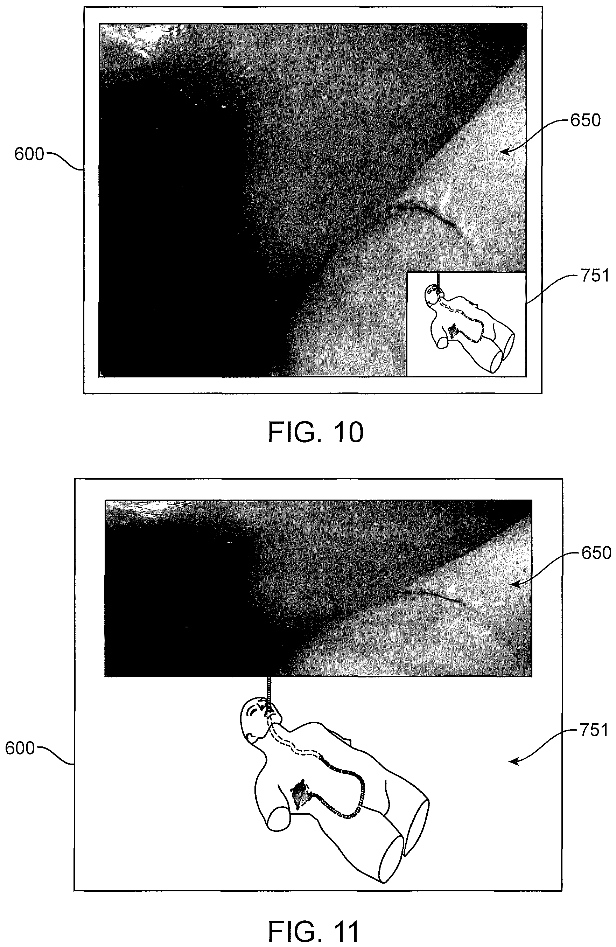

In addition to (or alternative of) displaying the patient and endoscope computer models, **720** and **710**, on the auxiliary display screen **160**, the computer models may be displayed on the primary display screen 600 along with the captured image **650**. For example, the oblique view **751** may be shown as a picture-in-picture insert to the captured image **650** on the primary display screen **600** as shown in **FIG. 10** or it may be shown in an adjacent window to that of the captured image **650** on the primary display screen **600** as shown in **FIG. 11**.

Referring back to **FIG. 4**, in **406**, a determination is made whether or not the operator has issued a stop guidance command. The operator may issue the stop guidance command in any one of a number of ways similar to, but complementary of those in which the operator issues the start guidance command.

If the determination in **406** is NO (i.e., navigation guidance mode has not been stopped by the operator), then the method loops back to **403** to update the current position and bend of the endoscope **110** and display a computer model of it along with the computer model of the patient for the next process cycle (or other programmed cycle). On the other hand, if the determination in **406** is YES (i.e., navigation guidance mode has been stopped by the operator), then the method proceeds to **407** in which the display processor **150** either causes the computer models of the patient and endoscope to no longer be displayed on the auxiliary display screen **160** and/or primary display screen **600** or freezes them on the display screen.

During the whole endoscopic procedure, starting from the insertion of the endoscope **110** in the patient's body, the proposed method, which is not part of the invention, stores in the memory device **155** digital versions of images periodically captured by the camera **141** (at the distal tip **112** of the endoscope **110**) together with the three-dimensional (3-D) position and orientation information of the camera **141** (as determined by the position processor **130**) with respect to the patient anatomy at the times (e.g., at a processing rate of 30 HZ) that the images were captured. Timestamps indicating the times are also stored in the memory device **155**. The resulting database provides endoscopic images tagged by their respective 3-D locations in the fixed reference frame that can be accessed at any successive time by the display processor **150** for the purpose of computing synthetic two-dimensional (2-D) and three-dimensional (3-D) views of the visited anatomy. Thus, a full recording of the endoscopic procedure may be stored in the memory device **155** (preferably a mass storage device, such as a hard drive, for this application) that may be played back on the display screens **600** and **160** for educational and/or archival purposes.

A first synthetic 2-D view can be computed by assembling (stitching together) multiple endoscopic images taken from neighboring 3-D locations into a single larger panoramic image according to the well known Computer Vision technique known as mosaicking (see for instance the seminal work by R. Szeliski, H.Y. Shum, Creating full view panoramic image mosaics and environment maps, In Proc. of ACM SIGGRAPH, 1997.) Endoscopic panoramas allow improved endoscopic navigation by providing a larger field of view then the endoscopic camera is capable of hence delivering greater awareness of the surrounding anatomy to the surgeon. Thus, the captured images shown in various figures herein on the primary display screen **600** are preferably panoramic images generated by the display processor **150** by mosaicking images retrieved from the memory device **155** which are representative of the most recently captured images by the camera **141**.

As an additional endoscopic navigation tool, graphical indications showing steering directions to previously defined landmarks in the patient are also provided as an aspect of the present invention.

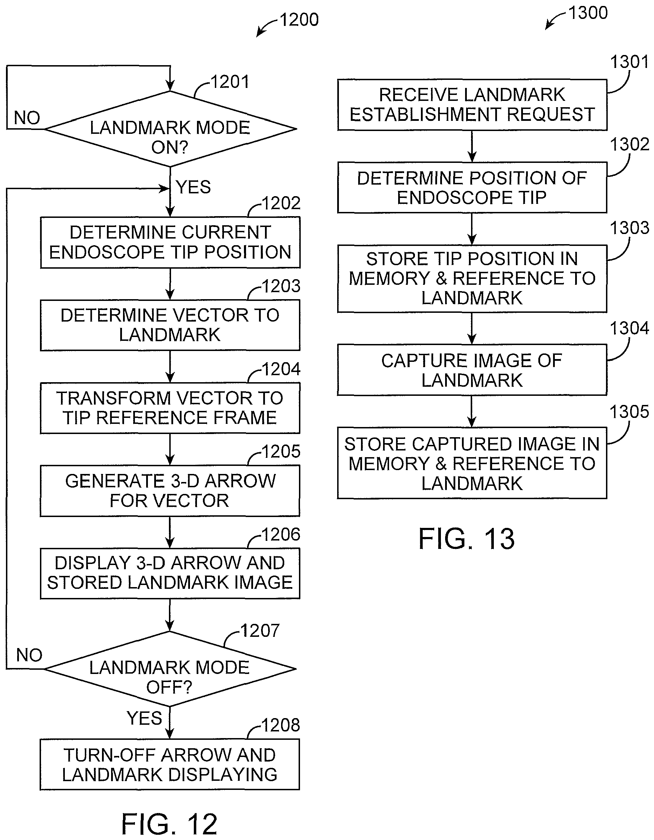

**FIG. 12** illustrates, as an example, a flow diagram of a computer implemented method **1200**, which is not part of the invention, for providing directional guidance to maneuver the steerable endoscope **110** to one or more landmarks in a patient. The computer in this case is preferably the one or more computers **200** that include the processors **130**, **140, 150**.

In **1201**, a determination is made whether or not the operator has turned on a landmarks directional guidance mode. The operator may turn on and/or off this mode in any one of a number of ways such as depressing an input button or flipping a switch on the input device **303** when the steerable endoscope **110** is being controlled teleoperatively, or depressing an input button or flipping a switch on the handle **116** when the steerable endoscope **110** is being controlled manually. Other ways may include voice actuation using a microphone and voice recognition processor (not shown) that may be implemented in or operatively coupled to the one or more computers **200**; a foot pedal (not shown) that may be depressed by the operator so as to turn on or off the mode; and a graphical user interface (GUI) **202** using the display screen **600** that the operator may interact with using an input device **201** as shown in **FIG. 2** to turn on and off the mode as well as specify other aspects or parameters of the method as described herein.

If the determination in **1201** is NO (i.e., landmark directional guidance is turned off), then the method loops through **1201** each process cycle (or other programmed cycle) until a YES determination is made, at which time, the method proceeds to **1202**.

In **1202**, the current position of the endoscope tip **112** in a fixed reference frame (i.e., a reference frame that does not move during the performance of a medical procedure using the endoscope **110**) is determined by the position processor **130** using information received from the optical fibers configured with strain sensors (e.g., optical fiber **501** in **FIG. 5**) that have been inserted in the flexible body **114** of the endoscope **110**.

The current position and orientation of the endoscope tip **112** may be readily determined in the fixed reference frame (for example, as previously explained in reference to **403** of **FIG. 4**) from the current bending of the optical fibers as indicated by information received from their strain sensors since their lengths are known, their proximal ends are fixed to a known location (such as fiber **501** to base point **502** as shown in **FIG. 5**), and their distal ends extend to the endoscope's tip **112**.

In **1203,** a vector connecting the current position of the endoscope tip **112** to the position of each landmark to which guidance indications are to be provided is determined by the display processor **150** using the endoscope tip position determined in **1202** and landmark positions stored in the memory device **155**.

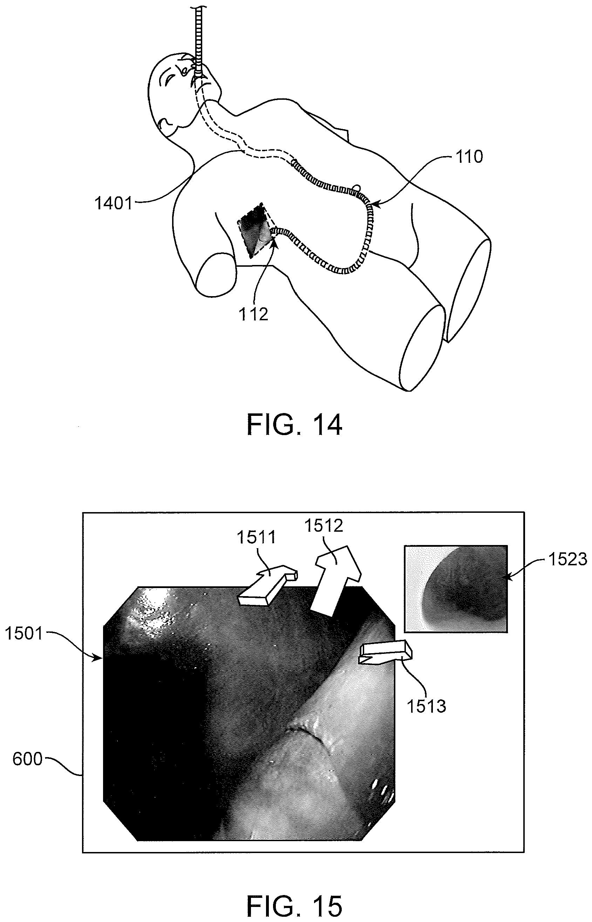

Typically, the landmarks are established by the operator as he or she guides the steerable endoscope **110** along its path from an entry point to a target site in the patient. As an example, **FIG. 14** shows an oblique view of a patient's head and torso where the steerable endoscope **110** has entered through the patient's mouth and its tip **112** guided to a target site. In this example, the endoscope **110** has been guided through the esophagus to the patient's stomach. The stomach was then entered and the endoscope **110** guided to the target site. Along the way, landmarks are established at the mouth entry and stomach entry using a method such as described below in reference to **FIG. 13**.

Alternatively, the positions of landmarks within the patient may be based upon preoperative measured data such as generated by Computer Axial Tomography (CAT), Magnetic Resonance Imaging (MRI), or X-rays. In this case, the patient's body (as he or she is lying down on the operating room table) is referenced to the fixed reference frame and the landmarks are registered with the patient's body so that their positions are determinable in the fixed reference frame by their respective positions in the patient's body. The landmark positions are then stored in the memory device **155** (or other storage device accessible to the display processor **150**). Although establishing landmarks in this manner may be time consuming and expensive, it allows the method described herein to provide directional guidance to landmarks in front of the endoscope tip **112** (i.e., between the current position of the endoscope tip and the target site), not just behind it (i.e., between the entry point into the patient and the current position of the endoscope tip).

In **1204**, the vectors determined in **1203** are then transformed from the fixed reference frame to a reference frame associated with the endoscope tip **112** (or camera **141**) by the display processor **150**.

In **1205**, 3-D arrows (or other graphical representations) indicative of the transformed vectors determined in **1204** are generated by the display processor **150**. The directions of the 3-D arrows are referenced to the endoscope tip's reference frame so that they correspond to directions that the operator should steer the endoscope's tip **112** using the handle or electromechanical interface **116** as previously described.

The sizes of the 3-D arrows, on the other hand, may be generated so as to be proportional to the magnitudes of the translated vectors so that landmarks further away are represented by larger arrows. As an alternative, the sizes may be generated so as to be inversely proportional to the magnitudes of the translated vectors so that landmarks further away are represented by smaller arrows. As yet another alternative, the sizes may be generated so as to each have the same length (such as shown by arrows **1511**, **1512** and **1513** in **FIG. 15**).

The 3-D arrows may also be color-coded so that the color of each arrow is uniquely associated with a particular anatomic structure. For example, referring to **FIG. 15**, a 3-D arrow **1511** indicating a steering direction towards the mouth entry landmark may be colored red, a 3-D arrow **1512** indicating a steering direction towards the stomach entry landmark may be colored yellow, and 3-D arrow **1513** indicating a steering direction towards the colon entry landmark may be colored blue. Alternatively, the edges of the display screen **600** may be colored to correspond to landmarks. The color schemes used herein may be operator defined or correspond to the color scheme used on MR viewers.

In **1206**, the 3-D arrows (or other graphical representations) generated in **1205** and a current image (as currently captured at the endoscope tip **112** and processed by the image processor **140** or a panoramic image as previously described using mosaicking of the current and previously captured images) are displayed on the primary display screen **600** by the display processor **150**. In addition, previously captured images of the landmarks may also be selectively or automatically retrieved from the memory device **155** and displayed adjacent or otherwise proximate to their respective 3-D arrows. The positions of the 3-D arrows may be displayed on the display screen **600** so as to relate to the orientations of the translated vectors so that, for example, arrows pointing to the left are positioned on the left side of the image and arrows pointing to the right are positioned on the right side of the image. Alternatively, the 3-D arrows may all be positioned near the same side of the image or spaced around the image. In the case where edges of the display screen **600** may be colored to correspond to landmarks, the 3-D arrow corresponding to each landmark is displayed adjacent its respective colored edge so that the association of the 3-D arrow and landmark is clear.

In addition to showing the landmarks as 3-D arrows on or near the current endoscope image on the primary display screen **600**, the landmarks can be displayed together with the 3-D patient and endoscope computer models being displayed on the auxiliary display screen **160** wherein positions of stored landmarks may be shown in their proper locations relative to the patient computer model. The landmarks may be indicated, for example, by a text label with the landmark name, a thumbnail image with the stored endoscope image captured with the landmark, a small graphic icon related to the type of landmark, or with an approximate 3-D model of the landmark.

Selection of which, if any, landmark images are to be displayed may be made by the operator, for example, using the input device **201** and GUI **202** to select one display option from a menu including: all landmark images to be displayed, no landmark images displayed, and only nearest landmark image to be displayed. Alternatively, the operator may not be provided an option and the choice pre-selected and programmed into the display processor **150**. Position, size, color and other options for displaying the 3-D arrows on the display screen **600** may be selected by the operator using the input device **201** and GUI **202** as shown in **FIG. 2**.

As an example of such displaying, **FIG. 15** shows the display screen **600** having a current image **1501** (i.e., an image currently captured by the endoscope **110** or a panoramic image as described elsewhere herein) displayed on it along with arrows **1511**, **1512** and **1513** respectively providing directional guidance to the mouth entry, stomach entry and colon entry landmarks. In addition, a previously captured image **1523** of the colon entry landmark is shown being displayed in a window adjacent to its associated arrow **1513**, which happens to be the nearest landmark to the current position of the endoscope tip **112**.

In **1207**, a determination is made whether or not the operator has turned off the landmark directional guidance mode. The operator may turn off the mode in any one of a number of ways similar to, but in an opposite manner of those in which the operator turned on the landmark guidance mode (e.g., placing a switch in an "off" vs. "on" position).

If the determination in **1207** is NO (i.e., landmark directional guidance mode has not been turned off by the operator), then the method loops back to **1202** to update the landmark directional vectors (e.g., **1511**, **1512**, **1513** in **FIG**. **15**) as the endoscope tip **112** is being guided to the target site and display updated graphical representations of the translated vectors on the display screen **600** along with a current image captured by the endoscope **110**. On the other hand, if the determination in **1207** is YES (i.e., landmark direction guidance mode has been turned off by the operator), then the method proceeds to **1208** in which the display processor **150** causes the graphical representations of the translated vectors and any corresponding landmark captured images to no longer be displayed on the display screen **600**.

It is to be appreciated that even though landmark directional guidance mode may be turned off, the operator may still be guiding the steerable endoscope **110** towards the target site and continually updated images captured at the endoscope tip **112** (and processed by image processor **130**) may still be displayed on the display screen **600** by the display processor **150**. For example, referring to **FIG. 15**, the display screen **600** may still have the current or panoramic image **1501** displayed on it, but the arrows **1511**, **1512**, **1513** and landmark image **1523** are no longer displayed after the operator turns off the landmark directional guidance mode.

It is also to be appreciated that the operator may interactively establish landmarks for which directional guidance is to be provided at any time while guiding the steerable endoscope **110** towards the target site. In particular, landmark establishment may be performed while landmark directional guidance mode is turned on as well as off.

**FIG. 13** illustrates, as an example, a computer implemented method **1300** for establishing anatomic structure landmarks as the steerable endoscope **110** is being guided through a patient's body. As in the case of the method **1200** described in reference to **FIG. 12**, the computer in this case is preferably the one or more computers **200** that include the processors **130**, **140**, **150**. In particular, it is preferably performed by the display processor **150** concurrently with other activities performed by the display processor **150** as described herein.

In **1301**, a landmark establishment request is received from the operator. The operator may initiate the request in any one of various well known interactive techniques including those previously described in reference to turning on and off the landmark directional guidance mode (but preferably a different one so as not confuse the two actions).

In **1302**, the current position of the endoscope tip **112** in the fixed reference frame is determined. Note that if the landmark directional guidance mode is turned on, this action is the same as **1202** of method **1200** and therefore, it does not have to be performed since the result from **1202** may be used in this case (i.e., it does not have to be performed a second time or twice in the same process cycle).

In **1303**, the current position of the endoscope tip **112** is then stored in the memory device **155** and referenced as the position of the landmark whose establishment has been requested. In **1304**, a current image captured at the endoscope tip **112** is identified as an image of the landmark and in **1305**, information of the image is also stored in the memory device **155** and referenced as the image of the landmark whose establishment has been requested.

As may be appreciated in performing the method **1300**, the order in which actions **1302** and **1304** occur is not important. In fact, the image processor **140** may process captured images at a different rate than that in which the position processor **130** determines current positions of the endoscope tip **112**. Therefore, the two actions may differ in the time as well as in the order in which they may each occur. In any event, upon receiving the landmark establishment request in **1301**, the next determined position of the endoscope tip **112** is stored in the memory device **155** and the next captured image received from the image processor **130** is stored in the memory device **155** without concern about which one occurs first.

In addition to the establishment of landmarks while navigating the endoscope **110** towards a target site within the patient, anatomic structures (such as the esophagus, stomach, colon, etc.) may be measured using position information of the endoscope tip **112** as it moves from one end of the anatomic structure to the other. Information of the measured anatomic structure may than be used to align and size 3-D models or preoperative measured data of the anatomic structures to their proper positions inside the patient's body and display the structures together with the 3-D patient computer model on the auxiliary display screen **160**. As an example, **FIG. 14** shows how the measured positions of the mouth and stomach entry landmarks have been used, together with the intermediate shape of the endoscope, to align and size a 3-D model of the esophagus connecting the mouth to the stomach entry. The esophagus model is then displayable as a 3-D curved tube **1401**, together with the 3-D models of the patient and the endoscope **110** on the auxiliary display screen **160**.

Although the various aspects of the present invention have been described with respect to one or more preferred embodiments, it will be understood that the invention is entitled to full protection within the full scope of the appended claims.

## Claims

1. A system (100) comprising:
a display (600,160);
an input device (303);
an endoscopic device (110) having a flexible body (114), steerable tip (112), and electromechanical interface (116);
an image capturing device (141) positioned at a distal end of the steerable tip (112) of the endoscopic device (110) so as to capture images from a perspective of the distal end;
a memory device (155); and
one or more processors (130, 140, 150) adapted to control bending of the flexible body (114) and steering of the steerable tip (112) of the endoscopic device (110) in response to manipulation of the input device (303), said one or more processors being **characterized in that** they are further adapted to reference a computer model of a patient to a reference frame, determine a current position and shape of the endoscopic device (110) relative to the reference frame, generate a computer model of the endoscopic device (110) according to the determined current position and shape, capture a plurality of images with the image capturing device (141), store the plurality of captured images in the memory device (155), determine and store positions of the image capturing device (141) at the times of such image capturing and display the computer models of the endoscopic device (110) and patient on the display (600,160) and a synthetic view generated by assembling the plurality of captured images into a panoramic image by mosaicing the plurality of images together so as to provide endoscopic navigation assistance to an operator of the system (100).

2. The system (100) according to claim 1, further comprising:
one or more optical fibers (120) configured with strain sensors extending through the endoscopic device (110) so as to provide shape information of the endoscopic device (110).

3. The system (100) according to claim 1, further comprising:
one or more optical fibers (120) configured with strain sensors and attached to the endoscopic device (110) so as to provide position information of the endoscopic device (110).

4. The system (100) according to claim 1, wherein the one or more processors (130, 140, 150) is adapted to display the panoramic image in a first area on the display (600,160) and the computer models of the endoscopic device (110) and patient in a second area on the display (600,160).

5. The system (100) according to claim 1, wherein the one or more processors (130, 140, 150) is adapted to display the panoramic image on the display (600,160) and the computer models of the endoscopic device (110) and patient as a picture-in-picture over the panoramic image on the display (600,160).

6. The system (100) according to claim 1, wherein the one or more processors (130, 140, 150) is adapted to display the panoramic image so as to appear to project from a distal tip of the computer model of the endoscopic device (110) being displayed on the display (600,160).

## Patentansprüche

1. System (100), das Folgendes umfasst:
ein Display (600, 160);
eine Eingabevorrichtung (303);
eine endoskopische Vorrichtung (110) mit einem flexiblen Körper (114), einer lenkbaren Spitze (112) und einer elektromechanischen Schnittstelle (116);
eine Bilderfassungsvorrichtung (141), die an einem distalen Ende der lenkbaren Spitze (112) der endoskopischen Vorrichtung (110) positioniert ist, um Bilder aus einer Perspektive des distalen Endes aufzunehmen;
eine Speichervorrichtung (155); und
einen oder mehrere Prozessoren (130, 140, 150), ausgelegt zum Steuern des Biegens des flexiblen Körpers (114) und des Lenkens der lenkbaren Spitze (112) der endoskopischen Vorrichtung (110) als Reaktion auf eine Manipulation der Eingabevorrichtung (303), wobei die genannten ein oder mehreren Prozessoren **dadurch gekennzeichnet sind, dass** sie ferner ausgelegt sind zum Beziehen eines Computermodells eines Patienten auf einen Bezugsrahmen, Bestimmen einer aktuellen Position und Form der endoskopischen Vorrichtung (110) relativ zu dem Bezugsrahmen, Erzeugen eines Computermodells der endoskopischen Vorrichtung (110) gemäß der bestimmten aktuellen Position und Form, Erfassen einer Mehrzahl von Bildern mit der Bilderfassungsvorrichtung (141), Speichern der Mehrzahl von erfassten Bildern in der Speichervorrichtung (155), Bestimmen und Speichern von Positionen der Bilderfassungsvorrichtung (141) zu Zeiten einer solchen Bilderfassung und Anzeigen der Computermodelle der endoskopischen Vorrichtung (110) und des Patienten auf dem Display (600, 160) und einer synthetischen Ansicht, erzeugt durch Zusammenfügen der Mehrzahl von erfassten Bildern zu einem Panoramabild durch mosaikähnliches Zusammensetzen der Mehrzahl von Bildern, um eine endoskopische Navigationshilfe für einen Bediener des Systems (100) bereitzustellen.

2. System (100) nach Anspruch 1, das ferner Folgendes umfasst:
eine oder mehrere optische Fasern (120), die mit Dehnungssensoren konfiguriert sind, die sich durch die endoskopische Vorrichtung (110) erstrecken, um Forminformationen über die endoskopische Vorrichtung (110) bereitzustellen.

3. System (100) nach Anspruch 1, das ferner Folgendes umfasst:
eine oder mehrere optische Fasern (120), die mit Dehnungssensoren konfiguriert und an der endoskopischen Vorrichtung (110) angebracht sind, um Positionsinformationen über die endoskopische Vorrichtung (110) bereitzustellen.

4. System (100) nach Anspruch 1, wobei die ein oder mehreren Prozessoren (130, 140, 150) zum Anzeigen des Panoramabildes in einem ersten Bereich auf dem Display (600, 160) und der Computermodelle der endoskopischen Vorrichtung (110) und des Patienten in einem zweiten Bereich auf dem Display (600, 160) ausgelegt ist.

5. System (100) nach Anspruch 1, wobei die ein oder mehreren Prozessoren (130, 140, 150) zum Anzeigen des Panoramabildes auf dem Display (600, 160) und der Computermodelle der endoskopischen Vorrichtung (110) und des Patienten als Bild in Bild über dem Panoramabild auf dem Display (600, 160) ausgelegt sind.

6. System (100) nach Anspruch 1, wobei die ein oder mehreren Prozessoren (130, 140, 150) zum Anzeigen des Panoramabildes so ausgelegt ist, dass es von einer distalen Spitze des Computermodells der endoskopischen Vorrichtung (110) bei dessen Anzeige auf dem Display (600, 160) vorzustehen erscheint.

## Revendications

1. Système (100) comportant :
un affichage (600, 160) ;
un dispositif d'entrée (303) ;
un dispositif endoscopique (110) ayant un corps flexible (114), un embout dirigeable (112), et une interface électromécanique (116) ;
un dispositif de capture d'images (141) positionné au niveau d'une extrémité distale de l'embout dirigeable (112) du dispositif endoscopique (110) de manière à capturer des images d'un point de vue de l'extrémité distale ;
un dispositif de mémoire (155) ; et
un ou plusieurs processeurs (130, 140, 150) adaptés pour commander la flexion du corps flexible (114) et la direction de l'embout dirigeable (112) du dispositif endoscopique (110) en réponse à la manipulation du dispositif d'entrée (303), lesdits un ou plusieurs processeurs étant **caractérisés en ce qu'**ils sont par ailleurs adaptés pour référencer un modèle informatique d'un patient par rapport à un cadre de référence, déterminer une position et une forme en cours du dispositif endoscopique (110) par rapport au cadre de référence, générer un modèle informatique du dispositif endoscopique (110) en fonction de la position et de la forme en cours ayant été déterminées, capturer une pluralité d'images au moyen du dispositif de capture d'images (141), stocker la pluralité d'images capturées dans le dispositif de mémoire (155), déterminer et stocker les positions du dispositif de capture d'images (141) aux instants de cette capture d'images et afficher les modèles informatiques du dispositif endoscopique (110) et du patient sur l'affichage (600, 160) et une vue synthétique générée par l'assemblage de la pluralité d'images capturées en une image panoramique en réalisant une mosaïque à partir de la pluralité d'images ensemble de manière à fournir une assistance de navigation endoscopique à un opérateur du système (100).

2. Système (100) selon la revendication 1, comportant par ailleurs :
une ou plusieurs fibres optiques (120) configurées avec des capteurs de contrainte s'étendant au travers du dispositif endoscopique (110) de manière à fournir des informations de forme du dispositif endoscopique (110).

3. Système (100) selon la revendication 1, comportant par ailleurs :
une ou plusieurs fibres optiques (120) configurées avec des capteurs de contrainte et attachées au dispositif endoscopique (110) de manière à fournir des informations de position du dispositif endoscopique (110).

4. Système (100) selon la revendication 1, dans lequel lesdits un ou plusieurs processeurs (130, 140, 150) sont adaptés pour afficher l'image panoramique dans une première zone sur l'affichage (600, 160) et les modèles informatiques du dispositif endoscopique (110) et du patient dans une deuxième zone sur l'affichage (600, 160).

5. Système (100) selon la revendication 1, dans lequel lesdits un ou plusieurs processeurs (130, 140, 150) sont adaptés pour afficher l'image panoramique sur l'affichage (600, 160) et les modèles informatiques du dispositif endoscopique (110) et du patient sous la forme d'une incrustation d'image par-dessus l'image panoramique sur l'affichage (600, 160).

6. Système (100) selon la revendication 1, dans lequel lesdits un ou plusieurs processeurs (130, 140, 150) sont adaptés pour afficher l'image panoramique de manière à sembler se projeter en provenance d'une extrémité distale du modèle informatique du dispositif endoscopique (110) qui est affiché sur l'affichage (600, 160).
